# EUROPÄISCHE PATENTSCHRIFT

(11) **EP 3 537 119 B1**
(45) Veröffentlichungstag und Bekanntmachung des Hinweises auf die Patenterteilung: **28.09.2022**
(21) Anmeldenummer: 18160090.9
(22) Anmeldetag: 06.03.2018
(51) Int. Cl.: G01J 3/02, B01F 35/213, B01F 35/22, B01F 35/92, B01F 35/90

(54) **SYSTEM MIT EINEM SPEISENZUBEREITUNGSGERÄT UND EINEM SPEKTROMETER**
SYSTEM WITH A FOOD PREPARATION DEVICE AND A SPECTROMETER
SYSTÈME AVEC UN DISPOSITIF DE PRÉPARATION D'ALIMENTS ET UN SPECTROMÈTRE

(43) Veröffentlichungstag der Anmeldung: 11.09.2019
(73) Patentinhaber: Vorwerk & Co. Interholding GmbH, 42270 Wuppertal (DE)
(72) Erfinder: Frielinghaus, Robert, 44799 Bochum (DE); Koetz, Hendrik, 58300 Wetter (DE)
(74) Vertreter: Gille Hrabal Partnerschaftsgesellschaft mbB Patentanwälte

(56) Entgegenhaltungen:
- WO-A1-2017/051424
- WO-A1-2018/015951
- DE-U1- 29 810 164
- US-A1- 2007 188 753
- US-A1- 2015 327 742
- US-A1- 2016 067 866
- US-A1- 2017 038 305

## Beschreibung

Die Erfindung betrifft ein System und Verfahren mit einem Spektrometer und einer Steuerungseinrichtung, die so eingerichtet ist, dass durch eine von dem Spektrometer bereitgestellte Analyseinformation aus einer Analyse einer Speise oder eines Speisebestandteils ein Nährstoff, Nahrungsbestandteil, Inhaltsstoff oder Zustand identifiziert werden kann.

Bislang werden die Nährwerte und der Energiegehalt einer insbesondere selbst zubereiteten Speise generisch ermittelten Rezeptangaben entnommen oder mittels Abwiegen und Nährwerttabellen anhand der Zutaten händisch ermittelt. Die Informationen über die Nährwerte und den Energiegehalt einer Speise vor dem Verzehr sind daher ungenau oder aufwändig zu ermitteln. Zudem kann aufgrund von Veränderungsprozessen der Speise oder einzelner Speisebestandteile beim Kochen in der Regel nicht einmal von den Zutaten zuverlässig auf die zubereitete Speise geschlossen werden.

Besonders in Privathaushalten ist ein zunehmendes Interesse an gesunder Ernährung, an qualitativ hochwertigen Speisen und an einer genauen Kenntnis der tatsächlichen Nährwerte selbst zubereiteter Speisen zu beobachten.

Die Druckschrift US2017038305A1 offenbart eine Anlage zum Bierbrauen, bei der eine kugelförmige Sonde mit integriertem Spektrometer zum Messen eingesetzt wird. Der innere Aufbau des Spektrometers der Sonde umfasst einen optischen Filter, durch den die Messstrahlung auf einen Detektor gelangt.

Die Druckschrift WO2017051424A1 offenbart einen Mixer mit einem Spektrometer zur Speisenanalyse. Das Spektrometer umfasst einen Filter, Linsen und einen Detektor.

Die Druckschrift US2016067866A1 offenbart eine Kochmaschine mit einem Spektrometer.

Die Druckschrift WO2018015951A1 offenbart ein portables Spektrometer zur Messung von Speisen.

Die Druckschriften US2007188753A1 und DE29810164U1 offenbaren Vorrichtungen zum industriellen Mischen von Lebensmitteln, die ein Spektrometer umfassen.

Es ist Aufgabe der Erfindung, eine Lösung bereitzustellen, die den oben beschriebenen Problemen Rechnung trägt.

Zur Lösung der Aufgabe dienen ein System gemäß Anspruch 1 und ein Verfahren gemäß Anspruch 2. Vorteilhafte Ausführungsformen ergeben sich aus den Unteransprüchen.

Offenbart wird ein Spektrometer mit einer Spektralzerlegungseinrichtung und einem Strahlungsdetektor, die so eingerichtet sind, dass die Spektralzerlegungseinrichtung eine einfallende elektromagnetische Messstrahlung wellenlängenabhängig in Anteile zerlegen und der Strahlungsdetektor die Intensität mindestens eines dieser Anteile messen kann. Das Spektrometer ist so eingerichtet, dass das Spektrometer eine Analyseinformation aus der Analyse einer Speise oder eines Speisebestandteils an ein Speisenzubereitungsgerät übermittelt und/oder über eine Ausgabeeinrichtung dem Benutzer ausgibt.

Analyse einer Speise oder eines Speisebestandteils, also z.B. einer Zutat, bedeutet das Identifizieren und/oder Quantifizieren eines Speisebestanteils, eines Nährstoffes, eines Nahrungsbestandteils oder eines sonstigen Inhaltsstoffes der Speise oder des Speisebestandteils und/oder das Ermitteln des Energiegehaltes oder eines Zustands der Speise oder des Speisebestandteils. Zu den Nährstoffen gehören die Hauptnährstoffe, d.h. Eiweiß, Fett und Kohlenhydrate oder z.B. Vitamine. Nahrungsbestandteile sind beispielsweise Wasser oder Cholesterin. Ein sonstiger Inhaltsstoff kann z.B. Natriumnitrid (NaNO₂) sein. Ein Zustand kann ein Bräunungsgrad beim Garen sein. Der Energiegehalt wird in der Regel in der Einheit Kcal ausgegeben. Eine Speise kann allgemein fest oder flüssig sein. Auch ein Speisebestandteil kann für sich genommen eine Speise sein wie z.B. Milch oder Trinkwasser.

Eine Analyseinformation kann ein Messsignal des Strahlungsdetektors oder ein Analyseergebnis sein. Eine Analyseinformation kann eine analoge oder digitale Form haben.

Ein Speisenzubereitungsgerät kann eine Speise zubereiten, d.h. erhitzen, rühren, mischen und/oder zerkleinern. Ein Speisenzubereitungsgerät kann beispielsweise ein Ofen, ein Kochautomat oder eine elektrische Küchenmaschine insbesondere mit einem Werkzeug zum Mischen und/oder Zerkleinern sein, das optional auch erhitzen kann.

Durch ein Spektrometer, das eine Analyseinformation aus der Analyse einer Speise oder eines Speisebestandteils an ein Speisenzubereitungsgerät übermittelt, kann die Rezeptur und/oder Zubereitungsparameter des Speisenzubereitungsgerätes überwacht und dynamisch angepasst werden und zwar vor und während der Zubereitung der Speise. Bislang waren solche Informationen einem Speisenzubereitungsgerät unbekannt. Durch den Einsatz eines Spektrometers können Informationen dieser Art dem Speisenzubereitungsgerät nun zugänglich gemacht und dadurch ein besonders reproduzierbares Kocherergebnis ermöglicht werden. Der Einsatz einer großen Menge von Einzelsensoren jeweils zur Erfassung eines bestimmten Stoffes oder eines bestimmten Zustands wie z.B. Bräunungsgrad durch Kameratechnik, Alkoholkonzentrationen durch Refraktometer oder Wassergehalt durch differentielle Thermometrie kann entfallen.

Durch ein Spektrometer, das Analyseinformationen aus der Analyse einer Speise oder eines Speisebestandteils über eine Ausgabeeinrichtung an den Benutzer ausgibt, kann der Benutzer visuell eine genaue Information über den Nährgehalt, den Energiegehalt oder möglicherweise unerwünschte Inhaltsstoffe erhalten, die tatsächlich in den Zutaten oder der zum Verzehr bereiten Speise vorliegen. Ferner kann durch ein akustisches Signal der Benutzer direkt durch das Spektrometer darauf hingewiesen werden, wenn beispielsweise eine gewünschte Beschaffenheit der Speise während der Zubereitung erreicht ist. Schließlich kann sich der Benutzer alternativ oder ergänzend Analyseinformationen über eine Datenschnittstelle für eine eigene Analyse ausgeben lassen. Ein rezeptbasiertes Abschätzen der Nährstoffe und des Energiegehaltes mithilfe von Nährwert- und Kalorienübersichten kann entfallen und eine detaillierte Information über die tatsächlich vorliegende Speise oder Speisebestandteil kann erhalten werden.

In einer Ausführungsform ist das Spektrometer portabel. Portabel meint mit einer Hand tragbar. Vorzugsweise genügt für die Bedienung und Handhabung ebenfalls eine Hand.. Ein besonders komfortabler und einfacher Einsatz für den Benutzer kann so sichergestellt werden. Ferner kann eine besonders einfach und kompakt aufgebaute Halterung zur temporären Fixierung an einem Küchengerät oder Ofen dienen, um beispielsweise für eine Prozessüberwachung des Zubereitungsprozesses eingebunden zu werden. Insbesondere hat ein portables Spektrometer ein Gewicht von weniger als einem Kilogramm, bevorzugt weniger als einem halben Kilogramm, ganz besonders bevorzugt ein Gewicht von weniger als 300 g. Um besonders komfortabel in der Hand zu liegen, hat das portable Spektrometer vorzugsweise die Form einer Fernbedienung, d.h. länglich mit einer mindestens doppelt und/oder höchstens fünffachen Länge im Vergleich zur Breite und/oder einer Dicke, die höchstens der Hälfte oder einem Viertel der Breite entspricht. Alternativ oder ergänzend kann das portable Spektrometer eine abgerundete, kugelartige oder kugelförmige Gestalt aufweisen, um für ein Zuführen zu einer Speise besonders robust und raumsparend zu sein. Bevorzugt umfasst ein portables Spektrometer einen Akku zur Versorgung mit elektrischer Energie.

In einer Ausführungsform ist das Spektrometer eingekapselt. Eingekapselt meint feuchtigkeits- und temperaturbeständig ummantelt durch ein kapselartiges Gehäuse. Das Spektrometer kann dadurch einer Speise vor, während oder nach der Zubereitung zugeführt werden. Die Einkapselung reduziert das Risiko bei der Verwendung eines Akkus bei Zugabe des Spektrometers in eine Speise insbesondere während der Zubereitung.

In einer Ausführungsform ist ein kapselartiges Gehäuse zum Einkapseln des Spektrometers vorgesehen, das eine bioverträgliche Beschichtung oder Außenwand aufweist. Bioverträglich meint, dass ein Abrieb oder das Material der Beschichtung oder der Außenwand selber unbedenklich verzehrt werden können, d.h. ohne nennenswerte Gesundheitsrisiken. Ein Gesundheitsrisiko durch das Zugeben des Spektrometers in eine Speise vor, während oder nach der Zubereitung kann somit vermieden oder zumindest signifikant reduziert werden.

In einer Ausführungsform umfasst das Spektrometer eine Datenschnittstelle für ein drahtloses Übermitteln der Analyseinformation. Ein Übermitteln der Analyseinformation kann auf diese Weise besonders einfach, hygienisch und komfortabel auch dann erfolgen, wenn das Spektrometer vor, während oder nach der Zubereitung zu einer Speise zugeführt wird. Aber auch der Bedienkomfort bei der portablen Verwendung beispielsweise zum Analysieren einer Zutat in einer Schüssel auf dem Tisch fem von einem Speisenzubereitungsgerät kann so erhöht werden. Insbesondere unterstützt die Datenschnittstelle Bluetooth oder WLAN.

Erfindungsgemäß umfasst das Spektrometer eine Strahlquelle zum Emittieren einer Anregungsstrahlung. Anregungsstrahlung meint elektromagnetische Strahlung zum Einbringen von Energie in die zu analysierende Speise oder den Speisebestandteil. Bevorzugt ist eine breitbandige Strahlquelle, z.B. ein Globar, eine Quarzhalogenlampe oder Hg-Hochdrucklampe, mit einem mittels Referenzmessung oder Kalibrierung bekannten Spektrum vorgesehen. Alternativ oder ergänzend wird ein Diodenlaser als Strahlquelle eingesetzt. Durch eine Strahlquelle kann eine besonders präzise Analyse erreicht werden.

Bei Vorsehen einer Strahlquelle trifft die Anregungsstrahlung, d.h. Photonen, der Strahlquelle auf die zu analysierende Speise. Dort werden die Moleküle durch diese eintreffende oder eingebrachte Energie angeregt. In Abhängigkeit von der Speise, dessen Eigenschaften oder Zustand wird die Anregungsstrahlung zu bestimmten Anteilen der eintreffenden Anregungsstrahlung durch die Speise reflektiert, absorbiert oder transmittiert. Insbesondere wird die Reflektionsstrahlung und/oder die Transmissionsstrahlung als die durch den Strahlungsdetektor zu erfassende Messstrahlung genutzt. Bevorzugt wird die Reflektionsstrahlung als die Messstrahlung genutzt. Mit Messstrahlung ist die für die Analyse der Speise zu analysierende Strahlung der Speise oder eines Speisebestandteils gemeint.

Vorzugsweise emittiert die Strahlquelle eine Anregungsstrahlung im Infrarotbereich, besonders bevorzugt im nahen Infrarotbereich (NIR). Eine Speise oder Speisebestandteiie können dadurch besonders zuverlässig analysiert werden. Erfindungsgemäß umfasst die Infrarot-Strahlung nahe Infrarotstrahlung (NIR) mit einem Wellenlängenbereich von 780 nm bis 1.400 nm und kurzwellige Infrarotstrahlung (SWIR) im Bereich von 1.400 nm bis 3.000 nm, insbesondere auch im mittlere Infrarotstrahlung (MWIR) im Bereich von 3.000 nm bis 8.000 nm, langwellige Infrarotstrahlung (LWIR) im Bereich von 8.000 nm bis 15.000 nm oder ferne Infrarotstrahlung (FIR) im Bereich von 15.000 nm bis 1.000.000 nm. Eine besonders große Anzahl von analysierbaren Stoffen und Zuständen kann so ermöglicht werden.

In einer Ausführungsform sind die Spektralzerlegungseinrichtung und der Strahlungsdetektor so eingerichtet, dass Messstrahlung Im Infrarotbereich oder nahen Infrarotbereich für die Analyse erfasst werden kann. Eine Speise kann so besonders zuverlässig analysiert werden.

Ein Strahlungsdetektor ist eine bevorzugt elektronische oder optoelektronische Einrichtung zum Messen der Intensität einer einfallenden elektromagnetischen Strahlung, also der Messstrahlung oder eines Anteils der Messstrahlung. Durch den Strahlungsdetektor wird ein zu der Intensität eines Anteils der Messstrahlung korrelierendes Messsignal bereitgestellt. Messstrahlung ist die elektromagnetische Strahlung von einem Objekt, also einer Speise oder eines Speisebestandteils, die zur spektroskopischen Analyse vorgesehen ist.

Eine Spektralzerlegungseinrichtung ist eine bevorzugt optische Einrichtung, die eine elektromagnetische Messstrahlung je nach Wellenlänge insbesondere durch Dispersion und/oder Brechung in eine unterschiedliche Richtung zu lenken oder zu leiten vermag, so dass ein erster Anteil der Messstrahlung mit einer ersten Wellenlänge auf eine erste Stelle des Strahlungsdetektors trifft und ein zweiter Anteil der Messstrahlung mit einer von der erste Wellenlänge unterschiedlichen zweiten Wellenlänge auf eine zweite Stelle des Strahlungsdetektors trifft. In einer Ausführungsform ist die Spektralzerlegungseinrichtung ein Prisma. Dies ermöglicht eine Spektralzerlegung mit besonders geringem Bauraum und Herstellungsaufwand. Alternativ oder ergänzend kann ein Gitter oder ein Interferometer als Spektralzerlegungseinrichtung vorgesehen werden.

Erfindungsgemäß umfasst der Strahlungsdetektor mindestens zwei getrennte Detektoreinheiten, insbesondere höchstens dreißig, bevorzugt höchstens fünfzehn, getrennte Detektoreinheiten. Ist entgegen der Erfindung nur eine Detektoreinheit umfasst, ist bevorzugt ein CCD-Sensor als Detektoreinheit vorgesehen, um mit besonders geringem Herstellungsaufwand einen besonders breiten Wellenlängenbereich abzudecken. Ein CCD-Sensor weist eine Matrix aus Pixeln auf, wobei jeder Pixel die Intensität einer einfallenden elektromagnetischen Strahlung oder eines Anteils der Messstrahlung in ein zu der Intensität korrelierendes Messsignal umzuwandeln vermag. Insbesondere sind die Pixel rechteckig, quadratisch oder polygonal vorzugsweise mit einer Kantenlänge von mindestens 1 µm und/oder höchstens 50 µm. Bevorzugt wird die Matrix aus Pixeln durch Mikrostrukturierung einer Metall-Isolator-Halbleiter-Schichtanordnung hergestellt. Sind mindestens zwei getrennte Detektoreinheiten von dem Strahlungsdetektor umfasst, also keine Pixel eines CCD-Sensors, wird für jede der Detektoreinheiten jeweils eine eigenständige Photodiode, die also unabhängig von einer anderen Photodiode positionierbar und fixierbar ist, eingesetzt. Eine Photodiode vermag die Intensität einer einfallenden elektromagnetischen Strahlung oder eines Anteils der Messstrahlung in ein zu der Intensität korrelierendes Messsignal umzuwandeln. Alternativ Ist es auch möglich, dass nur eine Photodiode eingesetzt wird, wenn nur eine Detektoreinheit vorgesehen ist, wobei dann die eine Photodiode entlang der wellenlängenabhängig abgelenkten Messstrahlung gefahren wird, so dass eine gemessene Intensität über die Position der Messung einer Wellenlänge zugeordnet werden kann.

In einer Ausführungsform wird eine Photodiode oder ein CCD-Sensor eingesetzt, der Infrarotstrahlung (IR), bevorzugt Nahinfrarotstrahlung (NIR), erfassen kann. Erfassen bedeutet, dass eine Intensität einer einfallenden elektromagnetischen Strahlung in ein zu der Intensität korrelierendes Messsignal umwandelt werden kann. Eine besonders präzise Analyse einer Speise oder eines Speisebestandteils wird so erzielt. In einer Ausführungsform wird als das Halbleitermaterial für die Photodiode oder den CCD-Sensor Quecksilber-Cadmium-Tellurid eingesetzt, um Messstrahlung im NIR-, SWIR-, MWIR- und LWIR-Bereich erfassen zu können. Alternativ oder ergänzend wird Indiumgalliumarsenid oder Blei(11)-sulfid eingesetzt, um Messstrahlung im NIR- und SWIR-Bereich erfassen zu können.

Erfindungsgemäß sind getrennte Detektoreinheiten an solchen Stellen des Strahlungsdetektors positioniert, auf die jeweils ein Anteil der Messstrahlung trifft, dessen Wellenlänge in Verbindung mit einer Intensitätsschwelle oder einem Intensitätsbereich einen spektroskopischen Fingerabdruck für einen bestimmten Speisebestanteil, Nährstoff, Nahrungsbestandteil, Inhaltsstoff oder Zustand definiert. Ein besonders kompakter Aufbau, der mit besonders geringem Aufwand hergestellt werden kann und auf die Analyse einer Speise oder eines Speisebestandteils zugeschnitten ist, wird ermöglicht. Da beispielsweise die Hauptnährstoffe im Rahmen einer spektroskopischen Messung Peaks bei bestimmten Wellenlängen aufweisen, also einen spektroskopischen Fingerabdruck haben, kann durch ein Vergleich der Messsignale mit hinterlegten Referenzwerten oder durch Überwachung von wellenlängenabhängigen Intensitätsschwellen eine Zuordnung und Identifikation erfolgen.

In einer Ausführungsform umfasst das Spektrometer eine optische Faser oder eine optische Kupplungsschnittstelle zum Ankoppeln einer optischen Faser. Eine optische Faser vermag elektromagnetische Strahlung, insbesondere im NIR-Bereich, zu leiten. Die optische Faser, die bevorzugt aus Glas oder Kunststoff hergestellt ist, erlaubt ein Biegen. Durch den Einsatz einer optischen Faser kann das Spektrometer an einer beliebigen Position in der Nähe eines Speisenzubereitungsgeräts abgelegt oder an das Speisenzubereitungsgerät vorübergehend manuell mechanisch an einer Halterung befestigt werden, so dass von dort die optische Faser auf die Speise oder einen Speisenbestandteil gerichtet werden kann. Die optische Faser kann insbesondere durch eine Öffnung in einem Deckel eines Speisenzubereitungsraumes geführt werden oder an eine optische Kupplungsschnittstelle an einem Gefäß oder einem Werkzeug eines Speisenzubereitungsgerätes angekoppelt werden, um auf die Speise gerichtet zu werden. Vorzugsweise sind zwei optische Fasern vorgesehen, von denen jeweils eine optische Faser optisch leitend mit der Strahlquelle und dem Strahlungsdetektor verbunden ist. Es kann so gezielt angeregt und gemessen werden.

In einer Ausführungsform ist eine optische Weiche vorgesehen, die so beschaffen ist, dass die Anregungsstrahlung durch die optische Faser und eine zusätzliche optische Faser geleitet werden kann oder die Messstrahlung durch die optische Faser und eine zusätzliche optische Faser geleitet werden kann. Eine optische Weiche ist ein Y-Verteiler. Es kann somit entweder Messstrahlung von der optischen Faser und der zusätzlichen optischen Faser zu demselben Strahlungsdetektors geleitet werden oder Anregungsstrahlung von nur einer Strahlquelle durch die optische Faser und die zusätzliche optische Faser geleitet werden.

Mit einer optischen Weiche kann ermöglicht werden, dass eine zusätzliche optische Faser zusätzlich zu der optischen Faser an die Strahlquelle bzw. den Strahlungsdetektor angeschlossen oder angekoppelt werden kann. Eine Speise kann so an zwei verschiedenen Stellen analysiert werden. Ferner kann eine Speise innerhalb und eine Speise oder ein Speisenbestandteil außerhalb eines Speisenzubereitungsgerätes durch dieselbe Strahlquelle und/oder denselben Strahlungsdetektor analysiert werden.

In einer Ausführungsform umfasst das Spektrometer ein Analysefenster. Analysefenster meint ein Fenster in dem Gehäuse des Spektrometers, das überwiegend durchlässig für die Anregungsstrahlung und/oder für die Analyse vorgesehene Messstrahlung ist. Bevorzugt ist das Analysefenster aus Glas hergestellt und/oder bioverträglich.

Ein weiterer Aspekt der Offenbarung betrifft ein System mit einem Spektrometer und einer Steuerungseinrichtung. Insbesondere handelt es sich bei dem Spektrometer um das Spektrometer gemäß dem eingangs beschriebenen Aspekt der Erfindung. Die Steuerungseinrichtung ist so eingerichtet, dass durch eine von dem Spektrometer bereitgestellte Analyseinformation aus einer Analyse einer Speise oder eines Speisebestandteils ein Nährstoff, Nahrungsbestandteil, Inhaltsstoff oder Zustand identifiziert wird. Ein besonders hoher Automatisierungsgrad und Komfort werden so erzielt. In einer Ausführungsform befindet sich die Steuerungseinrichtung in dem Spektrometer zur Verarbeitung und optional zur Auswertung der Messsignale des Strahlungsdetektors. Das Spektrometer kann dadurch besonders eigenständig und unabhängig von anderen Geräten betrieben werden und dem Benutzer beispielsweise ein Analyseergebnis anzeigen. In einer alternativen oder ergänzenden Ausführungsform befindet sich die Steuerungseinrichtung in einem Speisezubereitungsgerät oder in einem über ein Netzwerk, insbesondere über das Internet erreichbaren Computer zur Auswertung der Analyseinformation wie z.B. ein noch nicht ausgewertetes Messsignal des Spektrometers. Ein derart entfernter, vorzugsweise über das Internet erreichbarer Computer wird nachfolgend auch Cloud-Computer genannt. Aufwendige Elektronik kann dadurch im Spektrometer eingespart werden. Das Spektrometer kann dadurch besonders leicht, handlich und mit geringem Herstellungsaufwand bereitgestellt werden. Bei einem Auslagern der Steuerungseinrichtung in einen Cloud-Computer werden in einer Ausführungsform spektrale Fingerabdrücke in Form von hinterlegten Referenzwerten und/oder Datensätzen zentral verwaltet, d.h. für mehr als einen Benutzer oder für mehr als ein Spektrometer oder System. Optional können auch Auswertealgorithmen zentral in der Cloud hinterlegt und verwaltet werden. Kontinuierliche Optimierungen und Erweiterungen durch den Hersteller werden so ermöglicht.

In einer Ausführungsform umfasst die Steuerungseinrichtung eine Prozessoreinheit und eine Speichereinheit. Die Steuerungseinrichtung ist insbesondere so beschaffen, dass mithilfe der Prozessoreinheit ein Verfahren ausgeführt werden kann, bevorzugt anhand eines in der Speichereinheit gespeicherten Computerprogramms oder Befehlen. Insbesondere umfasst ein Verfahren Schritte zur Durchführung eines Auswertealgorithmus. Insbesondere sind ein spektraler Fingerabdruck und/oder ein Auswertealgorithmus in der Steuerung hinterlegt, also in der Speichereinheit digital gespeichert. Durch die Steuerungseinrichtung können die insbesondere analogen und/oder digitalen Messsignale des Spektrometers und optional Analyseergebnisse des Spektrometers verarbeitet werden.

Bevorzugt wird eine Signalvorverarbeitung und/oder Signalkorrektur z.B. zum Kompensieren eines Temperatureinflusses oder zur Analog-Digital-Umwandlung durchgeführt, bevor die so erhaltenen vorverarbeiteten Messwerte mithilfe der hinterlegten Daten insbesondere unter Anwendung des vordefinierten Auswertealgorithmus analysiert werden. Insbesondere findet die Signalvorverarbeitung in dem Spektrometer statt.

Erfindungsgemäß wird zur Identifizierung eines Nährstoffs, Nahrungsbestandteils, Inhaltsstoffs oder Zustands ein spektraler Fingerabdruck genutzt, der in Form eines Datensatzes hinterlegt ist, und zwar in der Speichereinheit der Steuerungseinrichtung. Dieser Datensatz weist für eine oder mehrere vordefinierte Wellenlängen der Messstrahlung jeweils eine vordefinierte Intensitätsschwelle oder einen vordefinierten Intensitätsbereich auf. Zeigt ein Vergleich der Messstrahlung mit den Datensätzen, dass die Bedingungen eines bestimmten Datensatzes durch die Messstrahlung erfüllt werden, wird der Nährstoff, der Nahrungsbestandteil oder die Speise, der Inhaltsstoff oder der Zustand der Speise oder des Speisebestandteils des erkannten spektralen Fingerabdrucks bzw. des entsprechend zugeordneten Datensatzes als Analyseergebnis ausgegeben.

Insbesondere kann ein Analyseergebnis ein Bräunungsgrad, eine Alkoholkonzentration, ein Wassergehalt, ein Kakaoanteil in einer Schokolade, ein Glutenanteil in Mehl, ein Hauptnährstoffgehalt oder ein Energiegehalt sein. Das Analyseergebnis kann sowohl ein qualitatives Vorkommen eines Inhaltsstoffes als auch dessen quantitative Konzentration sein. Auch die Identifikation eines Speisebestandteils, z.B. "Apfel", ist möglich. Insbesondere kann eine besonders präzise Analyse durch einen Datenbankabgleich bzw. eine Berücksichtigung des Rezeptes oder der gekochten Speise erzielt werden, wobei diese Information beispielsweise durch eine Küchenmaschine oder ein Smartphone der Steuerungseinrichtung ausgegeben werden kann.

In einer Ausführungsform erhält die Steuerungseinrichtung Daten von dem Speisenzubereitungsgerät und/oder übermittelt Steuerungssignale an das Speisenzubereitungsgerät. Daten können eine Rezeptinformation, eine von dem Speisenzubereitungsgerät gemessene Temperatur der Speise oder in dem Speisezubereitungsraum des Speisenzubereitungsgeräts oder andere für das Speisenzubereitungsgerät verfügbare analoge oder digitale Daten sein. Übermitteln kann allgemein durch ein Kabel oder kabellos erfolgen. Ein Steuerungssignal dient dem Ansteuern einer Funktionskomponente wie einem Heizelement oder Drehmotor für das Werkzeug des Speisenzubereitungsgeräts. Durch ein Steuerungssignal kann grundsätzlich auch ein Computerprogramm des Speisenzubereitungsgeräts aktiviert werden. Durch das Erhalten von Daten des Speisenzubereitungsgeräts und/oder durch das Übermitteln eines Steuerungssignals kann eine besonders präzise Analyse einer Speise erzielt werden. Eine präzise Analyse der Speise kann z.B. durch Berücksichtigung des Rezeptes weiter verbessert werden. Eine Prozessüberwachung oder Prozessregelung des Kochprozesses des Speisenzubereitungsgerätes können ermöglicht werden.

Da beispielsweise während des Zubereitungsprozesses häufig erhöhte Temperaturen herrschen, die sich ebenfalls in einem Infrarotstrahlungsanteil widerspiegeln, ist in einer Ausführungsform eine Temperaturmessung vorgesehen, die bevorzugt durch einen auch für andere Zwecke vorhandenen Temperatursensor z.B. einer Küchenmaschine oder eines Ofens erfolgt. Der Einfluss der Temperatur kann dann z.B. über ihre Planck-Kurve im Rahmen der Signalverarbeitung herausgerechnet werden.

In einer Ausführungsform ist die Steuerungseinrichtung so eingerichtet, dass mithilfe der Analyseinformation ein durch das Speisenzubereitungsgerät durchgeführter Zubereitungsprozess einer Speise überwacht oder geregelt wird. Ein besonders reproduzierbares Kochergebnis und/oder eine besonders genaue Kenntnis des Nährwertes der tatsächlich zubereiteten Speise können so ermöglicht werden. Beispielsweise kann in einer Ausführungsform auf Basis der tatsächlich zugeführten Speisebestandteile vor der Zubereitung und/oder dem tatsächlichen Zustand der Speise während der Zubereitung eine Prozessüberwachung und Optimierung des Zubereitungsprozesses durch ein Regeln einzelner Kochparameter wie Temperatur und Zeit oder durch ein Auffordern des Benutzers zur Zugabe eines bestimmten Speisebestandteils wie z.B. Zucker oder Wasser erfolgen. Nach der Zubereitung können Hauptnährstoffe, d.h. Kohlenhydrate, Fett und Eiweiß, ermittelt und der Energiegehalt der zubereiteten Speise in Kilokalorie (kcal) berechnet werden, um diese mit einem hinterlegten Diätplan abzugleichen, der bevorzugt in dem Speisenzubereitungsgerät gespeichert ist. In einer Ausführungsform sind das Spektrometer und/oder eine Küchenmaschine mit einem Ofen für ein Austauschen von Daten verbunden, um geräteübergreifende Funktionen bereitstellen zu können.

Ein weiterer Aspekt der Erfindung betrifft ein Verfahren zum Analysieren einer Speise mit dem System gemäß dem Anspruch 1, wobei ein Spektrometer die Speise oder einen Speisebestandteil der Speise vor, während und/oder nach einem Zubereiten der Speise analysiert, wobei die Zubereitung der Speise durch ein Speisenzubereitungsgerät erfolgt und/oder das Spektrometer mit dem Speisenzubereitungsgerät für ein Übermitteln einer Information verbunden ist, also eine Information an das Speisenzubereitungsgerät sendet und/oder von dem Speisenzubereitungsgerät empfängt. Durch dieses Verfahren kann besonders komfortabel eine gewünschte Speise zubereitet werden. Es kann eine präzise Kenntnis über eine selbst zubereitete Speise erlangt und ein reproduzierbares Kochergebnis ermöglicht werden.

Das Spektrometer analysiert die Speise oder ein Speisebestandteil bzw. Bestandteil der Speise vor, während und/oder nach einem Zubereiten der Speise wobei die Zubereitung der Speise durch ein Speisenzubereitungsgerät mit einem Speisenzubereitungsraum sowie einem Heizelement zum Erhitzen einer Speise in dem Speisenzubereitungsraum und/oder einem Werkzeug zum Mischen und/oder Zerkleinern einer Speise in dem Speisenzubereitungsraum erfolgt.

Nachfolgend werden Ausführungsbeispiele der Erfindung anhand von Figuren näher erläutert. Offenbarte Merkmale können beliebig mit den beanspruchten Gegenständen kombiniert werden. Die beanspruchten Schutzbereiche sind nicht auf die Ausführungsbeispiele beschränkt.

Es zeigen:
- Figur 1:: schematische Darstellung eines eingekapselten Spektrometers;
- Figur 2:: schematische Darstellung eines Spektrometers, das mit einem Werkzeug eines Speisenzubereitungsgeräts optisch ankoppelbar ist;
- Figur 3:: schematische Darstellung eines Spektrometers mit flexibel einsetzbaren optischen Fasern;
- Figur 4:: schematische Darstellung eines Spektrometers zur Analyse einer mit einem Speisenzubereitungsgerät zubereiteten Speise.

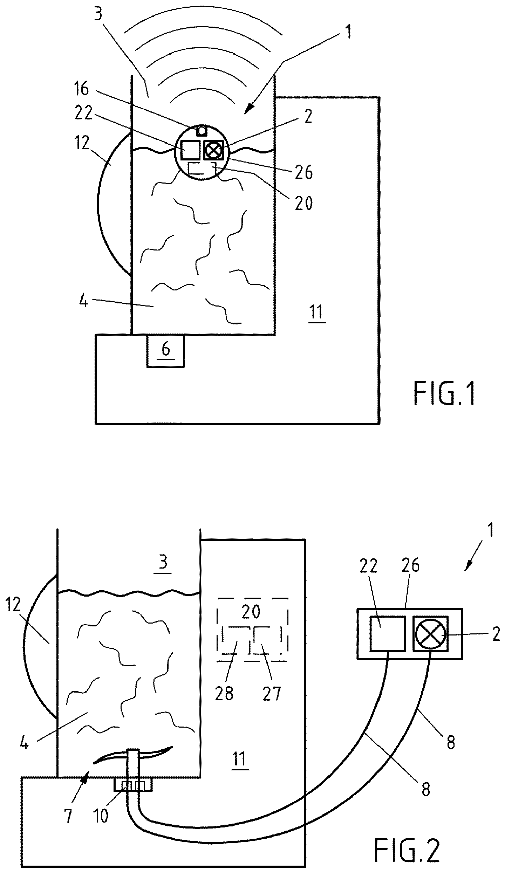

Die Figur 1 zeigt ein portables, durch ein wasserdichtes Gehäuse 26 eingekapseltes Spektrometer 1 mit einer Strahlquelle 2 und einem Strahlungsdetektor 22. Das gezeigte Spektrometer 1 vermag in einer Speise 4 mit niedriger Viskosität, z.B. eine Suppe, zu schwimmen, d.h. nicht abzusinken. Das Spektrometer 1 kann daher vor oder während der Zubereitung der Speise 4 in den Speisenzubereitungsraum 3 gemeinsam mit Zutaten eingebracht werden. Insbesondere ist bei flüssigen Speisen 4 vorgesehen, dass das Spektrometer 1 an, auf oder innerhalb der Speise schwimmt. Durch ein Heizelement 6 kann in Fig. 1 die Speise 4 erhitzt werden.

Eine Datenschnittstelle 16 ist vorgesehen, um wie in Fig. 1 dargestellt drahtlos Analyseinformationen an eine Cloud oder ein Speisezubereitungsgerät 11, 12 wie z.B. eine Küchenmaschine zu übermitteln, beispielsweise per Bluetooth oder WLAN.

Auch ein Austausch von Daten oder Informationen kann vorgesehen werden. Das Spektrometer 1 kann eine eigene Steuerungseinrichtung 20 umfassen, um eine Analyse vorzunehmen und ein Analyseergebnis auszugeben. Beispielsweise kann über einen integrierten Lautsprecher ein Warnsignal abgegeben werden, wenn ein vordefinierter und in der Speicherungseinrichtung 20 hinterlegter Zielzustand erreicht ist, wie z.B. ein Säuerungsgrad. Ein Messsignal und/oder Analyseergebnis können grundsätzlich auch über die Datenschnittstelle 16 ausgegeben werden. In Fig. 1 wird der Speisenzubereitungsraum 3 durch ein Gefäß 12 des Speisezubereitungsgeräts 11, 12 bereitgestellt, dessen Grundgerät 11 das Gefäß 12 aufnimmt. Insbesondere kann in dem Grundgerät 11 des Speisezubereitungsgeräts 11, 12 eine weitere Steuerungseinrichtung 20 integriert sein (in Fig. 1 nicht dargestellt). Das Spektrometer 1 sendet dann die Analyseinformation dorthin.

Die Figur 2 zeigt ein Spektrometer 1, bei dem die Strahlquelle 2 und der Strahlungsdetektor 22 jeweils mit optischen Fasern 8 verbunden werden können, um über eine Kupplungsschnittstelle 10 an ein Speisenzubereitungsgerät 11, 12 optisch angekoppelt und angeschlossen werden zu können. Insbesondere kann somit das Spektrometer Anregungsstrahlung durch ein Werkzeug 7 zum Mischen und/oder Zerkleinern in eine Speise 4 während der Zubereitung einbringen und daraus Messstrahlung für die Analyse der Speise 4 erhalten. Vorzugsweise kann das Speisenzubereitungsgerät 11, 12 mit dem Spektrometer 1 Daten austauschen, was in einer Ausgestaltung bei allen Systemen der Figuren 1 bis 4 umsetzbar ist. Eine dynamische Anpassung und/oder Regelung der Zubereitungsparameter wie z.B. Temperatur, Garzeit oder Drehzahl des Werkzeugs 7 können dadurch besonders zuverlässig realisiert werden und zwar in Abhängigkeit von den identifizierten Speisebestandteilen, den aktuell vorliegenden Inhaltsstoffen und des Ist-Zustandes der Speise. Ferner kann eine dynamische Anpassung der Zutatenliste während der Zubereitung bei Erkennung eines Mangels oder Überschusses eines speziellen Inhaltsstoffes vorgenommen und dem Benutzer angezeigt werden. Ein besonders optimiertes und/oder reproduzierbares Kochergebnis trotz schwankender Qualität und Quantität der anfangs zugeführten Speisebestandteile, also Nahrungsmittel bzw. Zutaten, sowie Veränderungen der Speisebestanteile während der Zubereitung beispielsweise durch Verdunstung oder Fermentierung kann so ermöglicht werden. Eine zerstörende oder speziell invasive Analyse kann entfallen.

Ein Datensatz für einen spektroskopischen Fingerabdruck, ein Auswertealgorithmus und/oder vordefinierte Überwachungsgrößen und Zielwert können in einer Speichereinheit 27 hinterlegt werden. Eine Prozessoreinheit 28 kann mithilfe dieser in der Speichereinheit hinterlegten Daten eine Signalvorverarbeitung und/oder Auswertung durchführen.

Ist ein Zielwert einer vordefinierten Überwachungsgröße erreicht, kann das Speisenzubereitungsgerät 11, 12 entsprechend vordefinierte Maßnahmen ergreifen, die bevorzugt ebenfalls in der Speichereinheit 27 gespeichert sind. Beispielhaft kann beim Garen oder Backen der Bräunungsgrad die Überwachungsgröße und der gewünschte Bräunungsgrad der Zielwert sein, so dass ein Heizelement 6 bei Erreichen des Zielwerts abgeschaltet oder heruntergeregelt wird. In einer Ausführungsform sind speisenspezifische oder benutzerspezifische Stopp- oder Abbruchkriterien definiert. Dadurch kann beispielsweise ein Garprozess angehalten werden, wenn durch die Analyse ein Erreichen eines vorbestimmten Bräunungsgrades ermittelt wurde. Dies kann beispielsweise bei der Destillation von Alkohol auch als Sicherheitsfunktion genutzt werden.

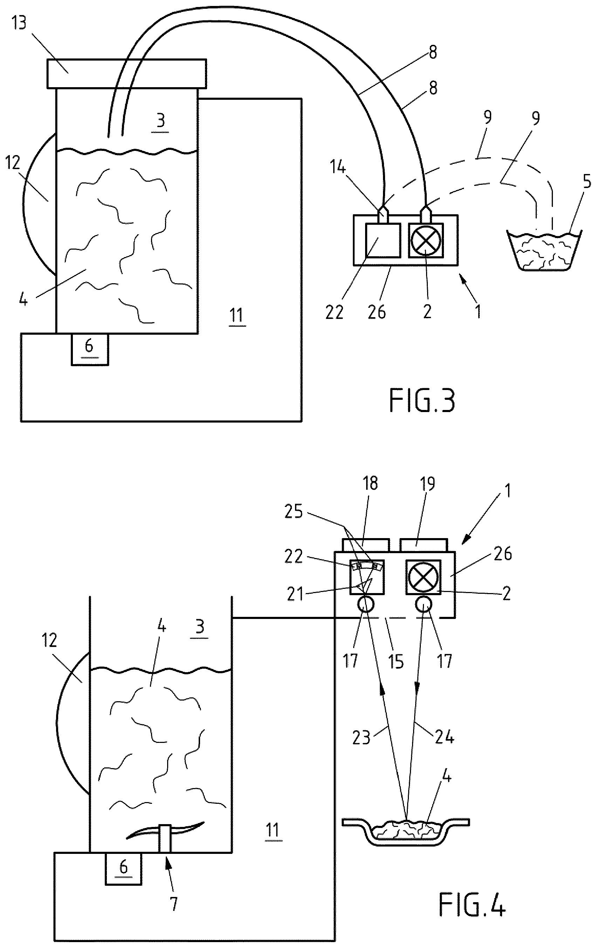

Die Figur 3 zeigt ein Spektrometer 1, bei dem optische Fasern 8 von dem Spektrometer 1 durch einen Deckel 13 in den Speisenzubereitungsraum 3 zur Analyse einer Speise vor, während oder nach der Zubereitung reichen. Mittels optischer Weichen 14 jeweils an der Strahlquelle 2 und dem Strahldetektor 22 sind parallel dazu jeweils zusätzliche optische Fasern 9 geschaltet, mit denen beispielsweise ein zuzuführender Speisebestandteil, also eine Zutat, analysiert werden kann, bevorzugt außerhalb des Speisenzubereitungsraums 3. Dadurch kann eine besonders genaue Kenntnis der tatsächlich verwendeten Zutaten bzw. zuzuführenden Speisebestandteile 5 und damit der Nährwert einer selbst zubereiteten Speise 4 z.B. für das Einhalten einer Diät erlangt werden. In einer Ausführungsform werden vor der Zubereitung die Speise 4 oder Speisebestandteile 5 in dem Speisenzubereitungsraum 3 analysiert. Der Kochprozess kann dadurch an schwankende Quantität oder Qualität des Ausgangszustands der Speise 4 vor der Zubereitung bzw. der eingeführten Speisebestandteile 5 angepasst werden, um selbst bei veränderten Mischungsverhältnissen, Rezeptveränderungen oder ohne Rezept zubereiteten Speisen ein reproduzierbares oder zumindest optimiertes Kochergebnis zu erhalten.

Die Figur 4 zeigt ein portables Spektrometer 1, insbesondere mit der Form und Größe ungefähr einer Fernseherfernbedienung. Das Spektrometer 1 ist mit nur einer Hand handhabbar und bedienbar. Ein Bedienschalter 18 ist neben einer Ausgabeeinrichtung 19 zum Ausgeben einer Analyseinformation angeordnet. Insbesondere ist die Ausgabeeinrichtung 19 eine Anzeige oder ein Touchscreen. Alternativ oder ergänzend kann die Ausgabeeinrichtung 19 eine SD Card Schnittstelle oder USB Schnittstelle sein. Das Spektrometer 1 ist zwischen 150 und 200 mm lang, zwischen 50 und 120 mm breit und zwischen 10 und 30 mm tief. Bevorzugt erfolgt nach einem Einschalten eine automatische Dunkel-Kalibrierung.

In einer Ausführungsform ist das Spektrometer 1 an einen Ofen, ein Gefäß 12, einen Deckel 13 oder ein Grundgerät 11 mechanisch ankoppelbar. Der Benutzer kann dann das Spektrometer 1 manuell mechanisch ankoppeln oder lösen. Im angekoppelten Zustand ist vorzugsweise eine Fixierung der Ausrichtung möglich, um auch in Abwesenheit des Benutzers einen Zubereitungsprozess mit dem Spektrometer 1 analysieren zu können. Eine Überwachung oder Regelung des Zubereitungsprozesses können dadurch besonders komfortabel mit dem portablen Spektrometer 1 umgesetzt werden.

In einer Ausführungsform ist eine Fokussier- und/oder Bewegungseinrichtung 17 vorgesehen, so dass eine Anregungsstrahlung 24 flexibel auf eine Speise 4 gerichtet werden kann und/oder eine Messstrahlung 23 von einer Speise 4 flexibel erfasst werden kann. Flexibel meint aus einer anderen Position, unter einem anderen Winkel oder mit einer anderen Fokuseinstellung oder Brennweite. Insbesondere ist die Bewegungseinrichtung ein motorisierter Umlenkspiegel. Insbesondere umfasst die Fokussiereinrichtung ein Linsensystem. Insbesondere ist an einem Eingang des Strahlungsdetektors 22 und/oder einem Ausgang der Strahlquelle 2 jeweils ein Analysefenster 15 vorgesehen. Bevorzugt ist die Fokussier- und/oder Bewegungseinrichtung 17 mit dem Eingang und/oder dem Ausgang optisch verbunden oder optisch angekoppelt.

In Figur 4 ist gezeigt, wie die Anregungsstrahlung 24 von der Strahlquelle 2 auf eine zubereitete Speise 4 gerichtet wird, die sich auf einem Teller befindet. Die Speise wiederum gibt eine Messstrahlung 23 ab, die ein reflektierter Anteil der Anregungsstrahlung 24 sein kann. Das Spektrometer 1 wiederum fängt diese Messstrahlung 23 ein. Mithilfe einer Spektralzerlegungseinrichtung 21 wird die Messstrahlung 23 in Abhängigkeit von der Wellenlänge in unterschiedliche Richtungen abgelenkt und fällt somit auf unterschiedliche Stellen des Strahlungsdetektors 22. Mehrere Detektoreinheiten 25 sind an solchen Stellen platziert, auf die ein Anteil der Messstrahlung 23 mit besonders großer Intensität oder einer Peak-Intensität trifft, wenn ein bestimmter Speisebestanteil, Nährstoff, Nahrungsbestandteil, Inhaltsstoff oder Zustand vorliegt.

In einer Ausführungsform wird dem Benutzer das Analyseergebnis durch eine Küchenmaschine und somit durch ein Speisenzubereitungsgerät 11, 12, ausgegeben. Beispielsweise kann vorgesehen werden, dass in Abhängigkeit von dem Analyseergebnis rezeptabhängig eine Empfehlung an den Benutzer ausgeben wird. Wenn beispielsweise wegen zu saurer Zutaten das geschmacklich empfohlene Zuckerniveau noch nicht erreicht ist, kann dem Benutzer ein Nachsüßen empfohlen werden. Alternativ oder ergänzend wird dem Benutzer das Analyseergebnis von dem Spektrometer 1 visuell, akustisch oder durch eine Übertragung auf ein externes Gerät wie z.B. Smartphone ausgegeben.

## Patentansprüche

1. System mit einem Speisenzubereitungsgerät (11, 12) und einem Spektrometer (1), das eine Strahlquelle (2) zum Emittieren einer Anregungsstrahlung umfasst und Reflexionsstrahlung als Messstrahlung nutzt,
wobei das System eine Steuerungseinrichtung (20) umfasst die so eingerichtet ist, dass durch eine von dem Spektrometer (1) bereitgestellte Analyseinformation aus einer Analyse einer Speise (4) oder eines Speisebestandteils (5) ein Nährstoff, Nahrungsbestandteil, Inhaltsstoff oder Zustand der Speise oder des Speisebestandteils identifiziert werden kann,
wobei das Spektrometer (1) mit einer Spektralzerlegungseinrichtung (21) und einem Strahlungsdetektor (22) ausgestattet ist, die so eingerichtet sind, dass die Spektralzerlegungseinrichtung (21) die einfallende elektromagnetische Messstrahlung (23) wellenlängenabhängig in Anteile zerlegt und der Strahlungsdetektor (22) die Intensitäten von mindestens zwei dieser Anteile misst, wobei das Spektrometer (1) so eingerichtet ist, dass das Spektrometer (1) eine Analyseinformation aus der Analyse einer Speise (4) oder eines Speisebestandteils (5) an das Speisenzubereitungsgerät (11, 12) übermitteln kann,
wobei der Strahlungsdetektor (22) mindestens zwei getrennte Detektoreinheiten (25) umfasst, wobei das Spektrometer (1) so eingerichtet ist, dass mithilfe der Spektralzerlegungseinrichtung (21) die Messstrahlung (23) in Abhängigkeit von der Wellenlänge in unterschiedliche Richtungen abgelenkt wird und die wellenlängenabhängigen Anteile der Messstrahlung (23) somit auf unterschiedliche Stellen des Strahlungsdetektors (22) fällt,
**dadurch gekennzeichnet, dass**
für jede der Detektoreinheiten jeweils eine eigenständige Photodiode eingesetzt ist, die also unabhängig von einer anderen Photodiode positionierbar und fixierbar ist;
die getrennten Detektoreinheiten (25) nur an solchen Stellen des Strahlungsdetektors (22) positioniert sind, auf die jeweils ein Anteil der Messstrahlung (23) trifft, dessen Wellenlänge in Verbindung mit einer Intensitätsschwelle oder einem Intensitätsbereich einen spektroskopischen Fingerabdruck für einen bestimmten Speisebestanteil, Nährstoff, Nahrungsbestandteil, Inhaltsstoff oder Zustand der Speise oder des Speisebestandteils definiert,
der in einer Speichereinheit (27) der Steuerungseinrichtung (20) digital in Form eines Datensatzes gespeichert ist, der für mehrere vordefinierte Wellenlängen der Messstrahlung (23) jeweils die vordefinierte Intensitätsschwelle oder den vordefinierten Intensitätsbereich aufweist, so dass der Nährstoff, der Nahrungsbestandteil oder die Speise, der Inhaltsstoff oder der Zustand der Speise oder des Speisebestandteils mittels des spektralen Fingerabdrucks bzw. des entsprechend zugeordneten Datensatzes als Analyseergebnis ausgegeben wird, nachdem erkannt wurde, dass ein Vergleich der detektierten Messstrahlung mit den Datensätzen gezeigt hat, dass die Bedingungen eines bestimmten Datensatzes durch die detektierte Messstrahlung erfüllt sind, und
dass auch solche Stellen des Strahlungsdetektors (22) vorhanden sind, durch die die mindestens zwei Detektoreinheiten (25) getrennt werden, und auf die jeweils ein Anteil der Messstrahlung (23) trifft, dessen Wellenlänge keinem in der Steuerungseinrichtung (20) gespeicherten spektroskopischen Fingerabdruck zugeordnet ist, und auf denen folglich keine Detektoreinheit (25) positioniert ist.

2. Verfahren zum Analysieren einer Speise (4) mit einem System nach dem vorhergehenden Anspruch, wobei das Spektrometer (1) des Systems die Speise (4) oder einen Speisebestandteil (5) der Speise (4) vor, während und/oder nach einem Zubereiten der Speise (4) analysiert, wobei das Spektrometer (1) mit dem Speisenzubereitungsgerät (11 , 12) des Systems für ein Übermitteln einer Information verbunden ist.

## Claims

1. System with a food preparation apparatus (11, 12) and a spectrometer (1) comprising a beam source (2) for emitting an excitation radiation and using reflection radiation as measurement radiation,
wherein the system comprises a control device (20) configured such that a nutrient, food constituent, ingredient or condition of a food or food component can be identified from an analysis of the food (4) or food component (5) by analysis information provided by the spectrometer (1),
wherein the spectrometer (1) is provided with a spectral decomposition device (21) and a radiation detector (22), which are configured such that the spectral decomposition device (21) decomposes the incident electromagnetic measurement radiation (23) into portions in a wavelength-dependent manner and the radiation detector (22) measures the intensities of at least two of these portions, wherein the spectrometer (1) is configured such that the spectrometer (1) can transmit analysis information from the analysis of a food (4) or a food component (5) to the food preparation apparatus (11, 12),
wherein the radiation detector (22) comprises at least two separate detector units (25), wherein the spectrometer (1) is configured such that by means of the spectral decomposition device (21) the measurement radiation (23) is deflected in different directions depending on the wavelength and the wavelength-dependent portions of the measurement radiation (23) thus fall on different locations of the radiation detector (22),
**characterized in that**
an independent photodiode is used for each of the detector units, which can thus be positioned and fixed independently of another photodiode;
the separate detector units (25) are positioned only at those locations of the radiation detector (22) which are each impinged upon by a portion of the measurement radiation (23) whose wavelength, in conjunction with an intensity threshold or intensity range, defines a spectroscopic fingerprint for a particular food component, nutrient, food constituent, ingredient or condition of the food or food constituent,
which is stored digitally in a memory unit (27) of the control device (20) in the form of a data set having the predefined intensity threshold or the predefined intensity range for a plurality of predefined wavelengths of the measurement radiation (23), respectively, so that the nutrient, the food constituent or the food, the ingredient or the condition of the food or the food component is output by means of the spectral fingerprint and/or the correspondingly assigned data set as an analysis result after it has been recognized that a comparison of the detected measurement radiation with the data sets has shown that the conditions of a particular data set are satisfied by the detected measurement radiation, and
**in that** there are also such locations of the radiation detector (22) by which the at least two detector units (25) are separated, and on which in each case a portion of the measurement radiation (23) impinges whose wavelength is not assigned to a spectroscopic fingerprint stored in the control device (20), and on which consequently no detector unit (25) is positioned.

2. Method for analyzing a food (4) with a system according to the preceding claim, wherein the spectrometer (1) of the system analyzes the food (4) or a food component (5) of the food (4) before, during and/or after a preparation of the food (4), wherein the spectrometer (1) is connected to the food preparation apparatus (11, 12) of the system for transmitting information.

## Revendications

1. Système comprenant un appareil de préparation d'aliments (11, 12) et un spectromètre (1) qui comprend une source de rayonnement (2) pour émettre un rayonnement d'excitation et utilise un rayonnement de réflexion comme rayonnement de mesure,
dans lequel le système comprend un dispositif de commande (20) qui est conçu de telle sorte que, par une information d'analyse fournie par le spectromètre (1) à partir d'une analyse d'un repas (4) ou d'un composant de repas (5), un nutriment, un composant alimentaire, un ingrédient ou un état du repas ou du composant de repas peut être identifié,
dans lequel le spectromètre (1) est équipé d'un dispositif de décomposition spectrale (21) et d'un détecteur de rayonnement (22) qui sont conçus de telle sorte que le dispositif de décomposition spectrale (21) décompose le rayonnement de mesure électromagnétique incident (23) en fractions en fonction de la longueur d'onde et que le détecteur de rayonnement (22) mesure les intensités d'au moins deux de ces fractions, dans lequel le spectromètre (1) est conçu de telle sorte que le spectromètre (1) puisse transmettre une information d'analyse provenant de l'analyse d'un repas (4) ou d'un composant de repas (5) à l'appareil de préparation d'aliments (11, 12),
dans lequel le détecteur de rayonnement (22) comprend au moins deux unités de détection séparées (25), dans lequel le spectromètre (1) est conçu de telle sorte qu'à l'aide du dispositif de décomposition spectrale (21), le rayonnement de mesure (23) est dévié dans différentes directions en fonction de la longueur d'onde et les fractions dépendantes de la longueur d'onde du rayonnement de mesure (23) tombent ainsi sur différents endroits du détecteur de rayonnement (22),
**caractérisé en ce que**
une photodiode indépendante est utilisée pour chacune des unités de détection, qui peut donc être positionnée et fixée indépendamment d'une autre photodiode ;
les unités de détection séparées (25) ne sont positionnées qu'aux endroits du détecteur de rayonnement (22) sur lesquels tombe respectivement une fraction du rayonnement de mesure (23) dont la longueur d'onde, en combinaison avec un seuil d'intensité ou une plage d'intensité, définit une empreinte spectrale pour un certain composant de repas, nutriment, composant alimentaire, ingrédient ou état du repas ou du composant de repas,
qui est mémorisé numériquement dans une unité de mémoire (27) du dispositif de commande (20) sous la forme d'un jeu de données qui présente, pour plusieurs longueurs d'onde prédéfinies du rayonnement de mesure (23), respectivement le seuil d'intensité prédéfini ou la plage d'intensité prédéfinie, de sorte que, moyennant l'empreinte spectrale ou bien le jeu de données associé, le nutriment, le composant alimentaire ou le repas, l'ingrédient ou l'état du repas ou du composant de repas est émis comme résultat d'analyse après qu'il a été reconnu qu'une comparaison du rayonnement de mesure détecté avec les jeux de données a montré que les conditions d'un certain jeu de données sont remplies par le rayonnement de mesure détecté, et
qu'il existe également des endroits du détecteur de rayonnement (22) par lesquels les au moins deux unités de détection (25) sont séparées et sur lesquels arrive respectivement une fraction du rayonnement de mesure (23) dont la longueur d'onde n'est associée à aucune empreinte spectrale mémorisée dans le dispositif de commande (20) et sur lesquels, par conséquent, aucune unité de détection (25) n'est positionnée.

2. Procédé d'analyse d'un repas (4) avec un système selon la revendication précédente, dans lequel le spectromètre (1) du système analyse le repas (4) ou un composant de repas (5) du repas (4) avant, pendant et/ou après une préparation du repas (4), dans lequel le spectromètre (1) est relié à l'appareil de préparation de repas (11, 12) du système pour une transmission d'une information.
